# EUROPEAN PATENT APPLICATION

(11) **EP 0 556 388 A1**
(43) Date of publication of application: **25.08.1993**
(21) Application number: 89911617.2
(22) Date of filing: 19.10.1989
(51) Int. Cl.: A61K 35/76, A61K 39/12, A61K 37/02

(54) **ANTIVIRAL AGENT**

(30) Priority: 21.10.1988 JP 265423/88
(71) Applicant: TORAY INDUSTRIES, INC., Tokyo 103 (JP)
(72) Inventor: TAMURA, Shinichi, Kanagawa-ken 238 (JP); KURATA, Takeshi, Tokyo 183 (JP); MORIYAMA, Masami 1561-1, Kudencho, Kanagawa-ken 247 (JP); NARUSE, Norio, Kanagawa-ken 248 (JP)
(74) Representative: Kador & Partner
(86) International application number: JP8901072
(87) International publication number: WO9004405

(57) **Abstract**

The invention provides an antiviral agent which contains as an active ingredient a conjugate between cholera toxin or its B subunit and a virus constituent. The virus constituent has a markedly enhanced antiviral activity owing to the conjugation with cholera toxin or its B subunit.

## Description

### [Technical Field]

The present invention relates to an antiviral agent comprising a viral structural component conjugated with cholera toxin (hereinafter referred to as "CT") or with cholera toxin subunit B (hereinafter referred to as "CTB").

### [Background Art]

Vaccines have been extensively used for prophylactic immunization against various diseases, giving good results. However, side reaction as well as insufficient effects have been encountered in many instances; hence a strong demand for improvement of such disadvantages. Attempts have been made to reduce the side reactions by further purifying a vaccine preparation or by adjusting its dose, but these measures may also lead to reduction of its immunizing potency.

Vaccines currently used in human comprise pathogen or a part thereof. The vaccines are inevitably contaminated with its constituents or growth factors, which will cause the side reactions upon vaccination. In addition, antigenic structure of the pathogen will activate immune system of the human body and cause various side reactions.

Furthermore, there exist many cases in which vaccination fails to provide an anticipated prophylactic effect, which requires improvement of the vaccine preparation itself.

### [Disclosure of Invention]

The present inventors have carried out extensive studies on methods to potentiate immunological properties of viral surface structural components (e.g. glycoproteins) without involving the use of vaccines. As the result, it was found that an antiviral effect of a viral structural component can be augmented by conjugating the component with CT or CTB, thus leading to this invention.

The present invention provides an antiviral agent comprising as an active ingredient a conjugate of a viral structural component with CT or CTB. The conjugation with CT or CTB affords a remarkable enhancement of the antiviral effect of the viral structural component.

The term "a viral structural component" in this specification means a nucleic acid or any other structural component which constitutes a virus. As the virus in this invention, viruses of Herpesviridae such as herpes simplex virus types I and II, varicella-zoster virus (VZV), cytomegarovirus (CMV), HHV-6 and HVV-6 as well as other viruses causing latent infection, human immunodeficiency virus (HIV) which is an etiologic agent of acquired immunodeficiency syndrome, and non-A non-B hepatitis virus may be used.

CT or CTB used in the present invention may be prepared by known methods of preparing CT and CTB.

CT or CTB may be conjugated to the viral structural component in a ratio of 0.1 - 10 : 0.01 - 10 by weight.

To achieve their conjugation, condensing agents which form peptide bonds or crosslinking agents for proteins may be used. Such condensing agents include, for example, EDC [1-ethyl-3-(3-dimethylaminopropyl) carbodiimide], N-hydroxysuccinimide, 1-hydroxybenzotriazole, N,N'-carbonyldiimidazole, and N,N'-dicyclohexylcarbodiimide. As crosslinking agents, disuccinimidyl suberate (DSS) dithiobis(succinimidyl propionate) (DSP) and N-(ε-maleimidocaproyloxy) succinimide (EMCS) may be mentioned for example.

In preparing the conjugate according to the present invention, all operations thereof should be carried out aseptically and each material to be used must be sterily obtained. Furthermore, pyrogens and allergenic substances (e.g. contaminant proteins) other than the viral structural component have to be eliminated as perfectly as possible.

A dosage form of the antiviral agent of the present invention, which comprises the thus prepared conjugate as the active ingredient, is preferably a liquid type suitable for an intranasal administration (e.g. intranasal spray, drops, and topical application) and for an injection, but powder spray is also practicable for the intranasal administration. An appropriate dose is 5 to 50 µl for mice and, 0.1 to 0.5 ml for human in both the intranasal administration and injection, being optionally changed.

The present invention is further illustrated by the following non-limiting Example and Experiment.

### Example

### Preparation of a conjugate of a synthetic peptide of a surface glycoprotein gD (23 amino acids at N-terminal) of herpes simplex virus type I (HSV-1) with cholera toxin subunit B

A synthetic peptide of gD-1 was prepared according to the N-terminal amino acid sequence (23 amino acids) as described previously (Gene, 26 (1983), 307-312). It was bound to CTB (Sigma Chemical Company, St. Louis, MO, USA; molecular weight 11,677) using EDC hydrochloride as follows. CTB was dissolved in 0.15M phosphate buffered saline (pH 7.2) and the resultant solution was combined with a 10-fold molar quantity of the synthetic peptide in water. To this mixture, a 50- to 250-fold molar excess of EDC hydrochloride was added to initiate binding reaction. The reaction was carried out overnight at room temperature with stirring on a stirrer. The reaction mixture was then passed through a Sephadex G-25 column to eliminate unreacted fractions of low molecular weight of less than 5,000.

### Experiment

### Induction of immune response to HSV-1 by intranasal administration with a conjugate of a synthetic N-terminal peptide (23 amino acids) of HSV-1 surface glycoprotein gD and cholera toxin subunit B

### 1. Induction of immune response to HSV-1

Balb/c mice (6 weeks old, female) were subjected to intranasal administration of a 1-mg/ml solution (20 µl) of the conjugate of the synthetic peptide (SP) with CTB (SP = CTB) under anesthesia. Three other groups of mice intranasally received comparable volumes of solutions consisting of SP alone, CTB alone and a mixture of CTB and SP (SP + CTB), respectively, so as to serve as control groups. Each group consisted of 5 mice. Six days later, each animal was injected with 25 µl of ultraviolet ray-inactivated HSV-1 suspension (1 × 10⁸ PFU/ml) subcutaneously into the right foot pad to elicit a delayed-type hypersensitivity (DTH). The degree of intensity of DTH reaction was assessed by measuring the thickness of the injected foot pad at 24 hours post-injection.

Results are presented in Table 1. Significantly pronounced DTH reactions were observed in the mice administered with the SP = CTB. The data have demonstrated that a cell-mediated immune response to HSV-1 can be induced by the intranasal administration of SP = CTB.

**Table 1**

| Induction of immune response to HSV-1 by administration of SP = CTB | | | | |
|---|---|---|---|---|
| Expt. No. | Intranasal administration (Day 0) | | Eliciting Antigen (Day 6) | Pad Swelling Reaction (thickness × 1/100 mm ±S.D.) |
| | Material used | dose | | |
| 1 | SP = CTB | 20 µg/20 µl/mouse | UV-inactivated virus, | 16.8 ±4.1 |
| | | | 1 × 10⁸ PFU/ml; | |
| 2 | SP | " | 25 µl/foot pad | 3.8 ±1.7 |
| 3 | SP + CTB | " | | 7.3 ±1.5 |
| 4 | CTB | " | | 3.5 ±1.7 |
| 5 | - | " | | 2.8 ±3.0 |

### 2. Induction of immune response to gD-1

BDF1 mice (16 weeks old, female) were dosed intranasally with 50 µl of SP = CTB (1 mg/ml) under anesthesia. Seven days afterwards, the animals were subcutaneously injected with 25 µl of various antigens into the right foot pad, and their DTH reactions to the respective eliciting antigens were assessed by measurement of the foot pad swelling at 24 hours after injection. The eliciting antigens used were: UV-inactivated virus (1 × 10⁸ PFU/ml), gD-1 (400 µg/ml) purified by affinity column chromatography using a monoclonal antibody against gD, purified gD-1 adsorbed to aluminum hydroxide gel (alum) (400 µg gD-1/800 µg alum), and ovalbumin (OVA) adsorbed to alum (400 µg OVA/800 µg alum).

Results of the experiment, as shown in Table 2, indicate that the DTH reaction induced by the intranasal administration of SP = CTB was provoked specifically with the purified gD-1 but not with OVA, a nonspecific protein.

Thus, it is obvious that the immune response to HSV-1 induced by the intranasal administration of SP = CTB was due to the cell-mediated immune reaction to gD-1, which had been derived by SP.

**Table 2**

| Induction of immune response to gD-1 by administration of SP = CTB | | | | |
|---|---|---|---|---|
| Expt. No. | Intranasal administration (Day 0) | | Eliciting Antigen (Day 7) | Pad Swelling Response (thickness × 1/100 mm ±S.D.) |
| | Material used | dose | | |
| 6 | SP = CTB | 50 µg/50 µl/mouse | UV-inactivated virus | 9.8 ±1.1 |
| 7 | SP = CTB | " | gD-1 | 18.0 ±4.4 |
| 8 | SP = CTB | " | gD-1/alum | 37.4 ±7.0 |
| 9 | SP = CTB | " | OVA/alum | 2.4 ±1.3 |
| 10 | - | - | gD-1/alum | 10.5 ±4.5 |
| 11 | - | - | - | 1.4 ±1.7 |

## Claims

1. An antiviral agent comprising as an active ingredient a conjugate of a viral structural component with cholera toxin or its subunit B.
